# EUROPEAN PATENT APPLICATION

(11) **EP 3 912 580 A1**
(43) Date of publication of application: **24.11.2021**
(21) Application number: 21175379.3
(22) Date of filing: 21.05.2021
(51) Int. Cl.: A61B 18/12, A61B 18/14

(54) **INDEPENDENT CONTROL OF DUAL RF BIPOLAR ELECTROSURGERY**

(30) Priority: 21.05.2020 US 202063028012 P; 21.05.2020 US 202063028007 P; 21.05.2020 US 202063028009 P; 21.05.2020 US 202063028049 P; 12.05.2021 US 202117318041
(71) Applicant: Covidien LP, Mansfield, MA 02048 (US)
(72) Inventor: HECKEL, Donald, W, Boulder, CO 80301-3299 (US); TONN, Donald, L, Boulder, CO 80301-3299 (US); FAULKNER, William, D, Boulder, CO 80301 (US)
(74) Representative: Maschio, Antonio

(57) **Abstract**

An electrosurgical generator includes a first radio frequency source having a first power supply configured to output a first direct current waveform; a first radio frequency inverter coupled to the first power supply and configured to generate a first interrogation waveform and a first radio frequency waveform from the first direct current waveform; and a first controller configured to control the first radio frequency inverter to output the first radio frequency waveform based on a response of the first interrogation waveform. The generator also includes a second radio frequency source having a second power supply configured to output a second direct current waveform; a second radio frequency inverter coupled to the second power supply and configured to generate a second interrogation waveform simultaneously as the first interrogation waveform and a second radio frequency waveform simultaneously as the first radio frequency waveform; and a second controller configured to control the second radio frequency inverter to output the second radio frequency waveform based on a response of the second interrogation waveform.

## Description

### CROSS-REFERENCE TO RELATED APPLICATIONS

The present application claims the benefit of and priority to U.S. Provisional Patent Applications Nos. 63/028,012, 63/028,007, 63/028,009, and 63/028,049, each of which was filed on May 21, 2020. The entire contents of each of the foregoing applications are incorporated by reference herein.

### BACKGROUND

### Technical Field

The present disclosure relates to systems and methods for controlling an electrosurgical generator. In particular, the present disclosure relates to controlling a plurality of bipolar electrosurgical devices that deliver two separate bipolar radio frequency waveforms.

### Background of Related Art

Electrosurgery involves application of high radio frequency electrical current to a surgical site to cut, ablate, desiccate, or coagulate tissue. In monopolar electrosurgery, a source or active electrode delivers radio frequency alternating current from the electrosurgical generator to the targeted tissue. A patient return electrode is placed remotely from the active electrode to conduct the current back to the generator.

In bipolar electrosurgery, return and active electrodes are placed in close proximity to each other such that an electrical circuit is formed between the two electrodes (e.g., in the case of an electrosurgical forceps). In this manner, the applied electrical current is limited to the body tissue positioned between the electrodes. Accordingly, bipolar electrosurgery generally involves the use of instruments where it is desired to achieve a focused delivery of electrosurgical energy between two electrodes.

The current solution for dual site surgery, namely, using two electrosurgical instruments simultaneously, is generally to use two electrosurgical generators. This solution is inherently cumbersome and cost prohibitive. Thus, there is a need for independent control of the multiple ports of a single electrosurgical generator using a common return path.

### SUMMARY

The present disclosure provides an electrosurgical system including an electrosurgical generator having two radio frequency (RF) channels generated by two separate RF sources. Each of the sources includes a power supply configured to output DC power and an RF power inverter configured to output an RF waveform. The individual RF waveform is supplied to a corresponding bipolar instrument. Each of the sources is controlled by its own controller, each of which is coupled to a common clock source. The electrosurgical generator performs a wide band measurement of each source's discontinuous or continuous signal simultaneously detecting cross-conductance and cross-talk between the RF sources. As used herein, cross-conductance is an energy channel's current flowing through the opposite channel's contact impedance and cross-talk is radiated interference between energy channels inside the electrosurgical generator.

According to one embodiment of the present disclosure, an electrosurgical generator is disclosed. The generator includes a first radio frequency source having a first power supply configured to output a first direct current waveform; a first radio frequency inverter coupled to the first power supply and configured to generate a first interrogation waveform and a first radio frequency waveform from the first direct current waveform; and a first controller configured to control the first radio frequency inverter to output the first radio frequency waveform based on a response of the first interrogation waveform. The generator also includes a second radio frequency source having a second power supply configured to output a second direct current waveform; a second radio frequency inverter coupled to the second power supply and configured to generate a second interrogation waveform simultaneously as the first interrogation waveform and a second radio frequency waveform simultaneously as the first radio frequency waveform; and a second controller configured to control the second radio frequency inverter to output the second radio frequency waveform based on a response of the second interrogation waveform.

According to another embodiment of the present disclosure, an electrosurgical system is disclosed. The system includes a first bipolar electrosurgical instrument and a second bipolar electrosurgical instrument. The system also includes an electrosurgical generator having a first radio frequency source including: a first power supply configured to output a first direct current waveform; and a first radio frequency inverter coupled to the first power supply and the first bipolar electrosurgical instrument. The first radio frequency inverter is configured to generate a first interrogation waveform and a first radio frequency waveform from the first direct current waveform. The first radio frequency source also including a first controller configured to control the first radio frequency inverter to output the first radio frequency waveform based on a response of the first interrogation waveform. The generator also includes a second radio frequency source having: a second power supply configured to output a second direct current waveform; and a second radio frequency inverter coupled to the second power supply and the second bipolar electrosurgical instrument. The second radio frequency inverter is configured to generate a second interrogation waveform simultaneously as the first interrogation waveform and a second radio frequency waveform simultaneously as the first radio frequency waveform. The second radio frequency source also including a second controller configured to control the second radio frequency inverter to output the second radio frequency waveform based on a response of the second interrogation waveform.

According to one aspect of any of the above embodiments, each of the first interrogation waveform and the second interrogation waveform is a pulsed radio frequency waveform. The pulsed radio frequency waveform includes a plurality of pulses having a duration from about 10 µsec to about 1,000 µsec and having a repetition rate of from about 10 msec to about 50 msec.

According to another aspect of any of the above embodiments, the first radio frequency source further includes at least one first sensor configured to measure a first impedance based the first interrogation waveform. The first controller is further configured to determine contact between a first electrosurgical instrument coupled to the first radio frequency source and tissue based on a comparison of the first impedance to an open circuit threshold. The first controller is further configured to control the first radio frequency inverter to output the first radio frequency waveform based on the comparison.

According to a further aspect of any of the above embodiments, the second radio frequency source further includes at least one second sensor configured to measure a second impedance based on the second interrogation waveform. The second controller is configured to determine contact between a second electrosurgical instrument coupled to the second radio frequency source and tissue based on a comparison of the second impedance to an open circuit threshold. The second controller is further configured to control the second radio frequency inverter to output the second radio frequency waveform based on the comparison.

According to yet another aspect of any of the above embodiments, the generator further includes a clock source coupled to the first controller and the second controller and configured to synchronize operation of the first controller and the second controller.

### BRIEF DESCRIPTION OF THE DRAWINGS

The present disclosure may be understood by reference to the accompanying drawings, when considered in conjunction with the subsequent, detailed description, in which:
FIG. 1 is a perspective view of an electrosurgical system according to an embodiment of the present disclosure;
FIG. 2 is a front view of a dual RF source electrosurgical generator of FIG. 1 according to an embodiment of the present disclosure;
FIG. 3 is a schematic diagram of the electrosurgical generator of FIG. 1 according to an embodiment of the present disclosure;
FIG. 4 is a schematic diagram of a clock source coupled to a first controller of a first RF source and a second controller of a second control source the electrosurgical generator of FIG. 1 according to the present disclosure;
FIG. 5 is a frequency response plot of continuous RF waveforms generated by the electrosurgical generator of FIG. 1 according to the present disclosure;
FIG. 6 is a frequency response plot of discontinuous RF waveforms generated by the electrosurgical generator of FIG. 1 according to the present disclosure;
FIG. 7 is a flow chart of a method for operating the electrosurgical generator of FIG. 1 to detect tissue contact during automatic bipolar mode according to an embodiment of the present disclosure; and
FIG. 8 is a flow chart of a method for operating the electrosurgical generator of FIG. 1 to detect overcurrent and/or cross-conductance according to an embodiment of the present disclosure.

### DETAILED DESCRIPTION

Embodiments of the presently disclosed electrosurgical system are described in detail with reference to the drawings, in which like reference numerals designate identical or corresponding elements in each of the several views. As used herein the term "distal" refers to the portion of the surgical instrument coupled thereto that is closer to the patient, while the term "proximal" refers to the portion that is farther from the patient.

The term "application" may include a computer program designed to perform functions, tasks, or activities for the benefit of a user. Application may refer to, for example, software running locally or remotely, as a standalone program or in a web browser, or other software which would be understood by one skilled in the art to be an application. An application may run on a controller, or on a user device, including, for example, a mobile device, an IOT device, a server system, or any programmable logic device.

In the following description, well-known functions or constructions are not described in detail to avoid obscuring the present disclosure in unnecessary detail. Those skilled in the art will understand that the present disclosure may be adapted for use with either an endoscopic instrument, a laparoscopic instrument, or an open instrument. It should also be appreciated that different electrical and mechanical connections and other considerations may apply to each particular type of instrument.

An electrosurgical generator according to the present disclosure may be used in monopolar and/or bipolar electrosurgical procedures, including, for example, cutting, coagulation, ablation, and vessel sealing procedures. The generator may include a plurality of outputs for interfacing with various ultrasonic and electrosurgical instruments (e.g., ultrasonic dissectors and hemostats, monopolar instruments, return electrode pads, bipolar electrosurgical forceps, footswitches, etc.). Further, the generator may include electronic circuitry configured to generate radio frequency energy specifically suited for powering ultrasonic instruments and electrosurgical devices operating in various electrosurgical modes (e.g., cut, blend, coagulate, division with hemostasis, fulgurate, spray, etc.) and procedures (e.g., monopolar, bipolar, vessel sealing).

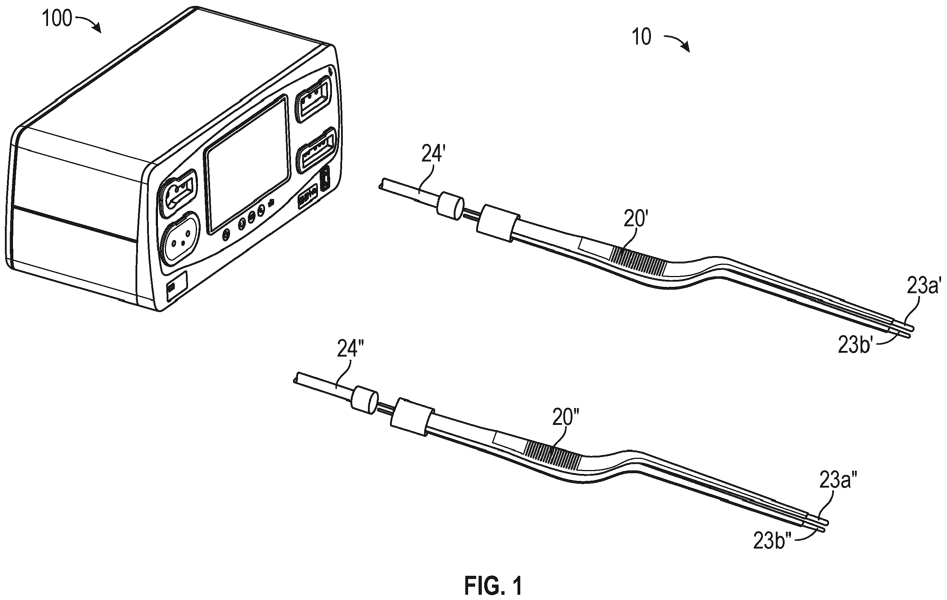

Referring to FIG. 1 an electrosurgical system 10 is shown which includes one or more bipolar electrosurgical instruments 20' and 20", which are shown as tweezers having a pair of electrodes 23a' and 23b' and 23a" and 23b", respectively, for treating tissue of a patient. In embodiments, the bipolar electrosurgical instruments 20' and 20" may be a pair of forceps. The instruments 20' and 20" are coupled to a generator 100 via cables 24' and 24". The generator 100 is a dual source RF generator configured to supply a separate RF waveform to each of the instruments 20' and 20" from individual RF sources.

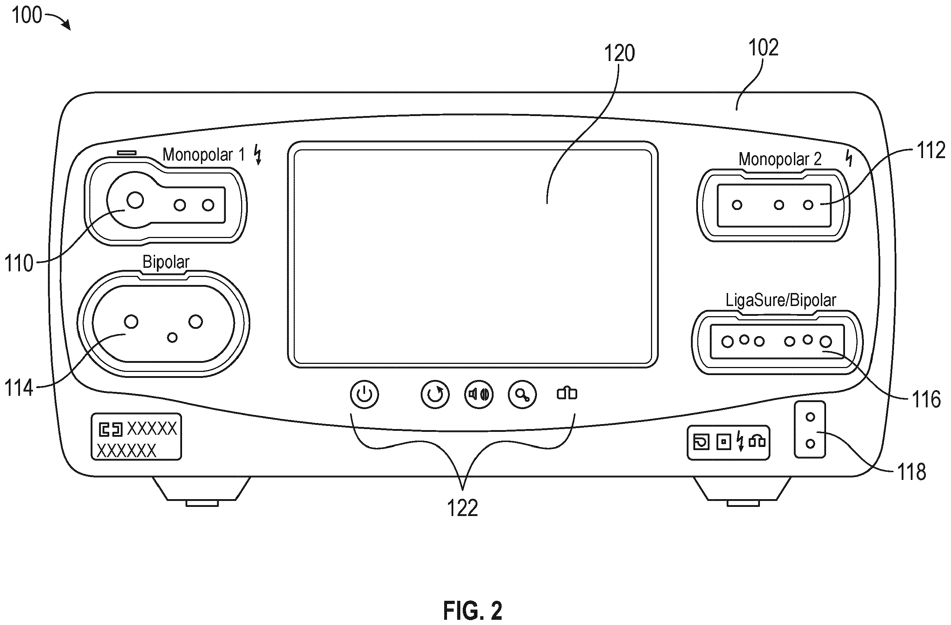

With reference to FIG. 2, a front face 102 of the generator 100 is shown. The generator 100 may include a plurality of ports 110, 112, 114, 116 to accommodate various types of electrosurgical instruments and a port 118 for coupling to a return electrode pad. The generator 100 includes a display 120 for providing the user with variety of output information (e.g., intensity settings, treatment complete indicators, etc.). The display 120 is a touchscreen configured to display a corresponding menu for the instruments (e.g., bipolar electrosurgical instruments 20' and 20", electrosurgical forceps, etc.). The user then adjusts inputs by simply touching corresponding menu options. The generator 100 also includes suitable input controls 122 (e.g., buttons, activators, switches, touch screen, etc.) for controlling the generator 100.

The generator 100 is configured to operate in a variety of modes and is configured to output bipolar waveforms, which is based on the selected mode. Each of the modes operates based on a preprogrammed power curve that dictates how much power is output by the generator 100 at varying impedance ranges of the load (e.g., tissue). Each of the power curves includes power, voltage and current control ranges that are defined by the user-selected intensity setting and the measured minimum impedance of the load.

The first and second RF waveforms may be either continuous or discontinuous and may have a carrier frequency from about 200 kHz to about 500 kHz. As used herein, continuous waveforms are waveforms that have a 100% duty cycle. In embodiments, continuous waveforms are used to impart a cutting effect on tissue. Conversely, discontinuous waveforms are waveforms that have a non-continuous duty cycle, e.g., below 100%. In embodiments, discontinuous waveforms are used to provide coagulation effects to tissue.

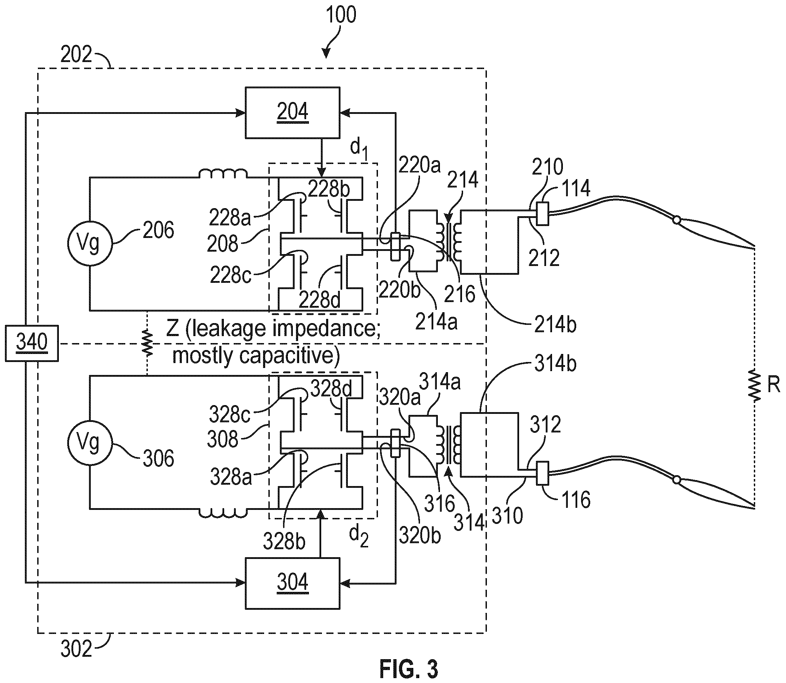

With reference to FIG. 3, the generator 100 includes a dual source RF architecture, where each RF source is supplied by an individual and separate RF inverter, each of which is powered by an individual and separate DC power supply. More specifically, the generator 100 includes a first RF source 202 and a second source 302. Each of the sources 202 and 302 includes a first controller 204 and a second controller 304, a first power supply 206 and a second power supply 306, and a first RF inverter 208 and a second RF inverter 308. The power supplies 206 and 306 may be high voltage, DC power supplies connected to a common AC source (e.g., line voltage) and provide high voltage, DC power to their respective RF inverters 208 and 308, which then convert DC power into a first and second RF waveforms through their respective active terminals 210 and 310. The energy is returned thereto via a first return terminal 212 and a second return terminal 312, respectively. In particular, electrosurgical energy for energizing the bipolar electrosurgical instruments 20' and 20" is delivered through the ports 114 and 116, each of which is coupled to the active terminal 210 and the return terminal 212 and the active terminal 310 and the return terminal 312, respectively.

The active terminal 210 and the return terminal 212 are coupled to the RF inverter 208 through an isolation transformer 214. The isolation transformer 214 includes a primary winding 214a coupled to the RF inverter 208 and a secondary winding 214b coupled to the active and return terminals 210 and 212. Similarly, the active terminal 310 and the return terminal 312 are coupled to the RF inverter 308 through an isolation transformer 314. The isolation transformer 314 includes a primary winding 314a coupled to the RF inverter 308 and a secondary winding 314b coupled to the active and return terminals 310 and 312.

The RF inverters 208 and 308 are configured to operate in a plurality of modes, during which the generator 100 outputs corresponding waveforms having specific duty cycles, peak voltages, crest factors, etc. It is envisioned that in other embodiments, the generator 100 may be based on other types of suitable power supply topologies. RF inverters 208 and 308 may be resonant RF amplifiers or non-resonant RF amplifiers, as shown. A non-resonant RF amplifier, as used herein, denotes an amplifier lacking any tuning components, i.e., conductors, capacitors, etc., disposed between the RF inverter and the load, e.g., tissue.

The controllers 204 and 304 may include a processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to perform the calculations and/or set of instructions described herein.

Each of the controllers 204 and 304 is operably connected to the respective power supplies 206 and 306 and/or RF inverters 208 and 308 allowing the processor to control the output of the first RF source 202 and the second source 302 of the generator 100 according to either open and/or closed control loop schemes. A closed loop control scheme is a feedback control loop, in which a plurality of sensors measures a variety of tissue and energy properties (e.g., tissue impedance, tissue temperature, output power, current and/or voltage, etc.), and provide feedback to each of the controllers 204 and 304. The controllers 204 and 304 then control their respective power supplies 206 and 306 and/or RF inverters 208 and 308, which adjust the DC and/or RF waveform, respectively.

The generator 100 according to the present disclosure may also include a plurality of sensors 216 and 316, each of which monitors output of the first RF source 202 and the second RF source 302 of the generator 100. The sensors 216 and 316 may be any suitable voltage, current, power, and impedance sensors. In the embodiment illustrated in FIG. 3, the sensors 216 are coupled to leads 220a and 220b of the RF inverter 208. The leads 220a and 220b couple the RF inverter 208 to the primary winding 214a of the transformer 214. The sensors 316 are coupled to leads 320a and 320b of the RF inverter 308. The leads 320a and 320b couple the RF inverter 308 to the primary winding 314a of the transformer 314. Thus, the sensors 216 and 316 are configured to sense voltage, current, and other electrical properties of energy supplied to the active terminals 210 and 310 and the return terminals 212 and 312.

In further embodiments, the sensors 216 and 316 may be coupled to the power supplies 206 and 306 and may be configured to sense properties of DC current supplied to the RF inverters 208 and 308. The controllers 204 and 304 also receive input signals from the display 120 and the input controls 122 of the generator 100 and/or the instruments 20' and 20". The controllers 204 and 304 adjust power outputted by the generator 100 and/or perform other control functions thereon in response to the input signals.

The RF inverters 208 and 308 includes a plurality of switching elements 228a-228d and 328a-328d, respectively, which are arranged in an H-bridge topology. In embodiments, RF inverters 208 and 308 may be configured according to any suitable topology including, but not limited to, half-bridge, full-bridge, push-pull, and the like. Suitable switching elements include voltage-controlled devices such as transistors, field-effect transistors (FETs), combinations thereof, and the like. In embodiments, the FETs may be formed from gallium nitride, aluminum nitride, boron nitride, silicone carbide, or any other suitable wide bandgap materials.

The controllers 204 and 304 are in communication with the respective RF inverters 208 and 308, and in particular, with the switching elements 228a-228d and 328a-328d. Controllers 204 and 304 are configured to output control signals, which may be pulse-width modulated ("PWM") signals, to switching elements 228a-228d and 328a-328d. In particular, controller 204 is configured to modulate a control signal d1 supplied to switching elements 228a-228d of the RF inverter 208 and the controller 304 is configured to modulate a control signal d2 supplied to switching elements 328a-328d of RF inverter 308. The control signals d1 and d2 provide PWM signals that operate the RF inverters 208 and 308 at their respective selected carrier frequency. Additionally, controller 204 and 304 are configured to calculate power characteristics of output of the first RF source 202 and the second source 302 of the generator 100, and control the output of the first RF source 202 and the second source 302 based at least in part on the measured power characteristics including, but not limited to, voltage, current, and power at the output of RF inverters 208 and 308.

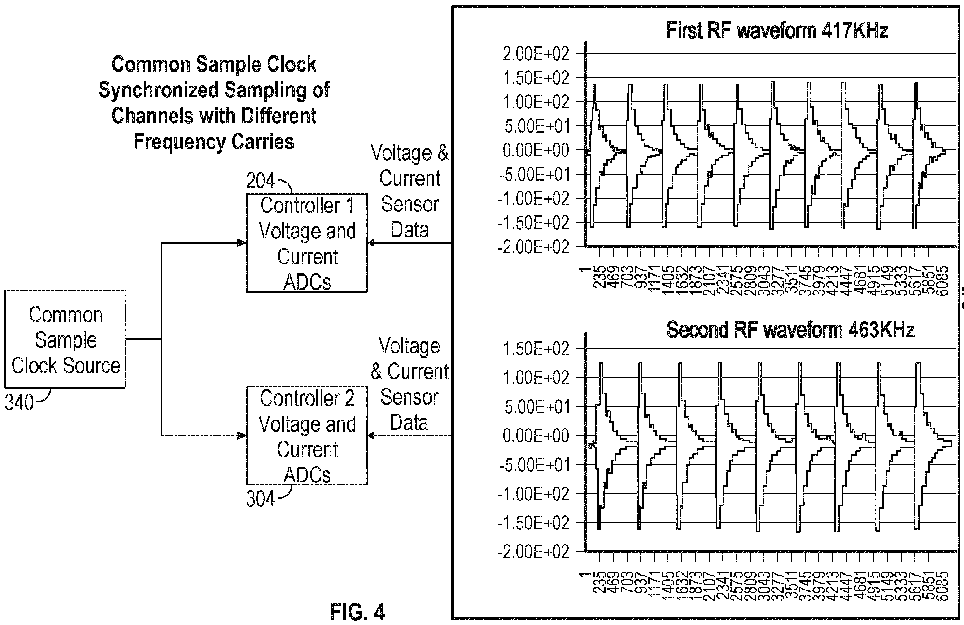

With reference to FIGS. 3 and 4, each of the controllers 204 and 304 is coupled a clock source 340 which acts as a common frequency source for each of the controllers 204 and 304, such that the controllers 204 and 304 are synced. The clock source 340 is an electronic oscillator circuit that produces a clock signal for synchronizing operation of the controllers 204 and 304. In particular, sampling operation of the controllers 204 and 304 is synchronized. Each of the controllers 204 and 304 generates an RF waveform based on clock signal from the clock source 340 and the selected mode. Thus, the once the user selects one of the electrosurgical modes, each of the controllers 204 and 304 outputs a first and second control signal, which are used to control the respective RF inverters 208 and 308 to output first and second RF waveforms corresponding to the selected mode. The selected mode for each of the first RF source 202 and the second source 302, and the corresponding RF waveforms, may be the same or different.

The RF waveforms have different carrier frequencies, such that the first RF waveform has a first carrier frequency and the second RF waveform has a second carrier frequency. The two different carrier frequencies are selected such that the controllers 204 and 304 can discriminate or separate measurement data in the frequency domain. The measurement data is collected by the sensors 216 and 316 which monitor the output of the first RF source 202 and the second source 302. The controllers 204 and 304 analyze their respective first and second RF waveforms using any suitable band pass technique or any technique which transforms the measurement data to the frequency domain, such as discrete Fourier transform (DFT) and fast Fourier transform (FFT). In embodiments, the controllers 204 and 304 may use arrays of Goertzel filters which are pointed to the carrier frequency and its harmonics of the RF waveform for continuous waveforms (e.g., those used during cut mode). With respect to discontinuous waveforms, the Goertzel filters are pointed to the repetition rate and harmonics of the repetition frequency of the waveform being analyzed. The filtering of measurement data may be performed by applications, e.g., software instructions, executable by the controllers 204 and 304.

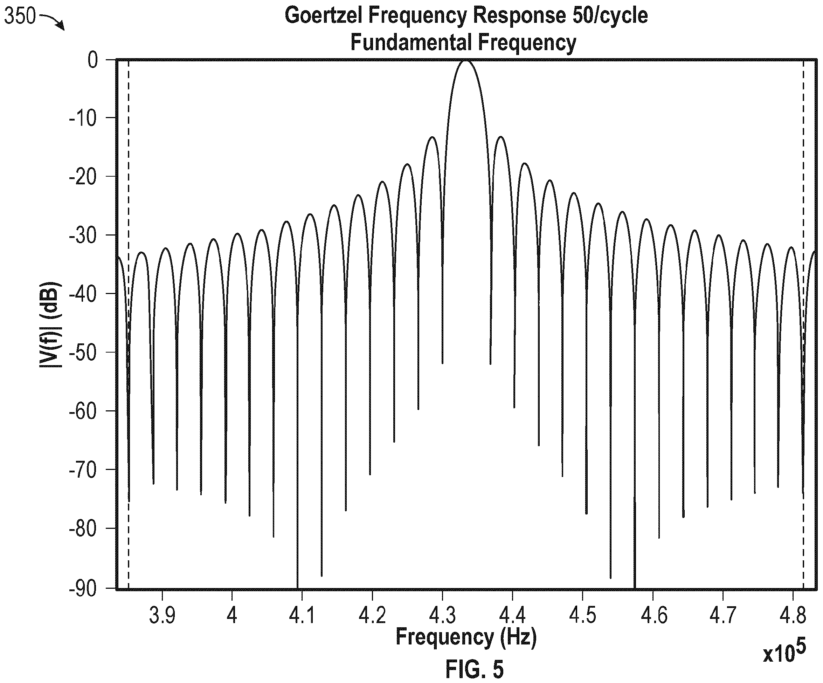

The frequencies for the first and second RF waveforms are selected to provide sufficient channel separation between the carrier frequencies of the first RF waveform and the second RF waveform as determined by the band pass Goertzel filters. With reference to a frequency response plot 350 of FIG. 5, the opposite RF port frequencies for continuous waveforms are chosen to be at the null points of the frequency response. This maximizes the source-to-source separation.

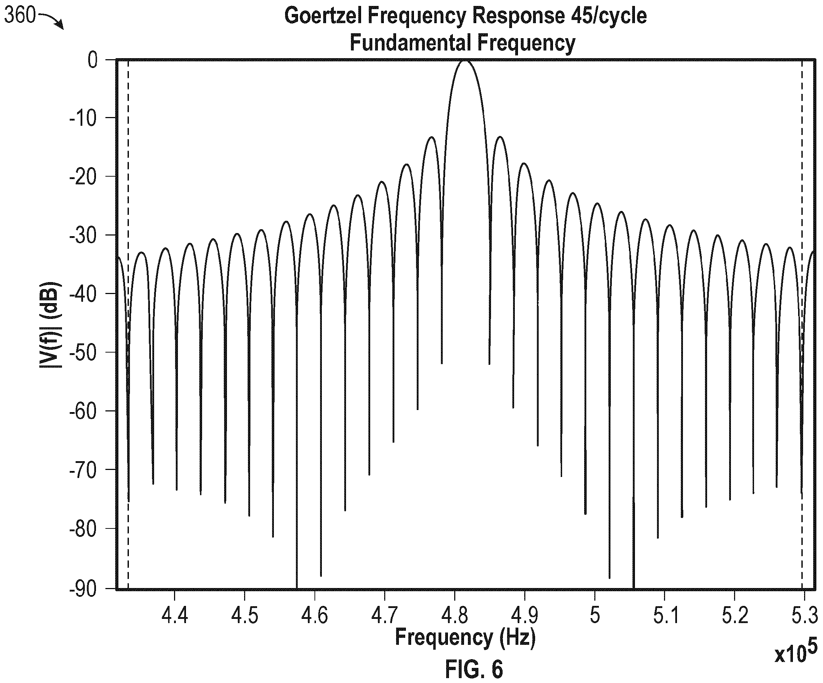

With reference to FIG. 6, which shows a frequency response plot 360 for discontinuous waveforms, a separate Goertzel filter is pointed at the base repetition rate as well as even and odd harmonics of the RF waveform being analyzed. As the fundamental frequencies are not completely orthogonal, there is overlap of certain harmonics. The overlap of sets of harmonics produce a discontinuity in the Goertzel array plot. This is used to detect if significant cross-conductance from one RF source is occurring in the sensors 216 or 316 of another RF source. If it is desired to separate the sources which include combined information in only 1 of a plurality of harmonics, the unimpacted harmonics are used in combination with a percentage of the impacted harmonics. The separated cross-conductance information is used as a dosage monitor to detect excessive cross-conductance situations.

In embodiments, where both the first and second RF waveforms are discontinuous, the limited frequency space may be constraining. Discontinuous waveforms may have a repetition rate from about 20 KHz to about 490 KHz. The repetition rate does not allow for a completely orthogonal solution from a signal processing standpoint. The technique for determining the power value for each of the first RF source 202 and the second source 302 to be used for independent control is dependent on the level of cross-conductance. Cross-conductance results in real power deposited at the contact impedance. Thus, the power to control is based on the sum of cross-conductance power deposited at the contact impedance site and port source power deposited at the contact impedance. In embodiments, power deposited in the non-contact tissue impedance and the return electrode pad 26 may also be included in the overall calibration of a specific RF port. If frequency discrimination is used for power control, prior to use the generator 100, the instruments 20' and 20", and the return electrode pad 26 are used in a calibration procedure, which takes into account both cable compensation for supply lines 24' and 24" and the return line 28 as well as harmonic overlap and cross-conductance. Cross-conductance may also be monitored as a mitigation to potential dosage errors.

In embodiments, where one the first and second RF waveforms is continuous while the other is discontinuous, the continuous RF waveform may be at the higher Goertzel frequency of about 481 KHz and the discontinuous RF waveform may be at a lower frequency of about 433 KHz, such that there is very little interference that happens between the sources due to the frequency response of the Goertzel being divisible by 45 (see FIGS. 5 and 6) as well as the concentration of discontinuous energy at or below its carrier frequency of 433 KHz.

In embodiments, where the first and second RF waveforms are continuous, since the continuous waveforms are generally not a perfect sine wave, the pre-selected unique carrier frequencies of 433 KHz and 481 KHz also comply with coherent sampling rules used to separate the continuous RF sources in combination with a Goertzel filter to provide attenuation of the interfering constructive or destructive signal from the other source. A band pass filter provides a signal pass-band region and lower / upper signal rejection regions. The level of rejection is variable and based on the type of band pass filter designed. Finite impulse response (FIR) filters provide a sinc function (sin x/x) type magnitude vs frequency response. The implementation of a computationally efficient Goertzel filter acts like a sampling function as shown in plots 350 and 360 of FIGS. 5 and 6.

The generator 100 is configured to operate in an automatic bipolar mode, during which each of the first RF source 202 and the RF second source 302 output any suitable bipolar RF waveform upon detection of tissue contact. The user may configure each of the first RF source 202 and the RF second source 302 during this mode to set the power level and set a delay time for commencing RF delivery after confirming that the bipolar electrosurgical instruments 20' and 20" are in contact with tissue. In this mode, the generator outputs a low power interrogation pulse waveform to measure impedance and determine contact with tissue. The interrogation waveform may be from about 1 W to about 5 W, and may have a duration from about 10 µsec to about 1,000 µsec, and may be repeated every 10 msec to about every 50 msec. Upon detecting an impedance indicative of tissue contact, the generator 100 outputs energy through the connected bipolar instruments 20' and 20" after a user-selectable delay time.

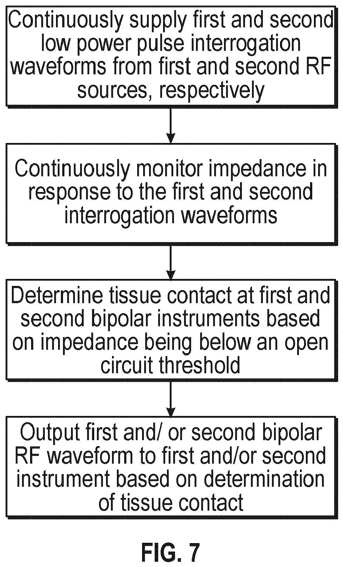

With reference to FIG. 7, a method for controlling the generator 100 is disclosed. The method provides for simultaneous dual activation of the first RF source 202 and the RF second source 302. Initially, each of the first RF source 202 and the second RF source 302 are configured by selecting an automatic bipolar mode. As described above, each mode is associated with a predetermined RF waveform, which may be continuous or discontinuous, and is based on the desired tissue effect. The auto bipolar mode RF waveform may be a discontinuous waveform. The user also configured individually each of the first RF source 202 and the second RF source 302 to include a power level of the RF waveform and a delay for generation of the RF waveform following detection of tissue contact.

During operation, each of the first RF source 202 and the RF second source 302 output a first interrogation waveform and a second interrogation waveform continuously while the mode is active. The first and second interrogation waveforms are segregated and are used by the sensors 216 and 316 to measure impedance of the tissue, if any, being contacted by each of the instruments 20' and 20". Each of the processors 204 and 304 continuously compares the impedance in response to the first and second interrogation waveforms to a predetermined threshold that is indicative of a resistive load being present (e.g., not an open circuit), which may be 1,000 Ω. In further embodiments, the impedance may be compared to a low threshold indicative of a short circuit which may be from about 10 Ohms (Q) to about 50 Ω.

The processors 204 and 304 utilize signal processing techniques and/or time-multiplexing techniques to discriminate between simultaneous activated first and second interrogation waveforms. The processors 204 and 304 also determine level of cross-conductance between the first RF source 202 and the second RF source 302. Interrogation waveform carrier frequencies are such that the carrier frequencies provide coherent sampling under time critical power computation update rates and to provide a combination of effective frequency discrimination as well as for detection of cross-conductance. Carrier frequency ratios may be varied between 45:50 Goertzel ratios to 49:51 Goertzel ratios to provide for different response rates for tissue contact detection, i.e., differentiating from an open circuit. Time cascaded Goertzel computations may also be used to provide a smaller interval of time between power computations for algorithm.

In embodiments, optimal frequencies for the first and second interrogation waveforms may be determined by scanning frequency response of the first RF source 202 and the second RF source 302 and identifying noise frequencies. Following identifying noise carrier frequencies, quieter frequencies are selected to avoid corruption of the first and second interrogation waveforms thereby avoiding false detection of tissue contact.

In further embodiments, pulses of the first and second interrogation waveforms may be separated in time such that they are not overlapping. Since the duration (e.g., from about 10 µsec to about 1,000 µsec) of each pulse of the first and second interrogation waveforms is smaller than the off time in between the pulses (e.g., from about 10 msec to about every 50 msec), then the pulses may be synchronized to any time slot within the other waveform's off period, such that the pulses do not occur at the same time.

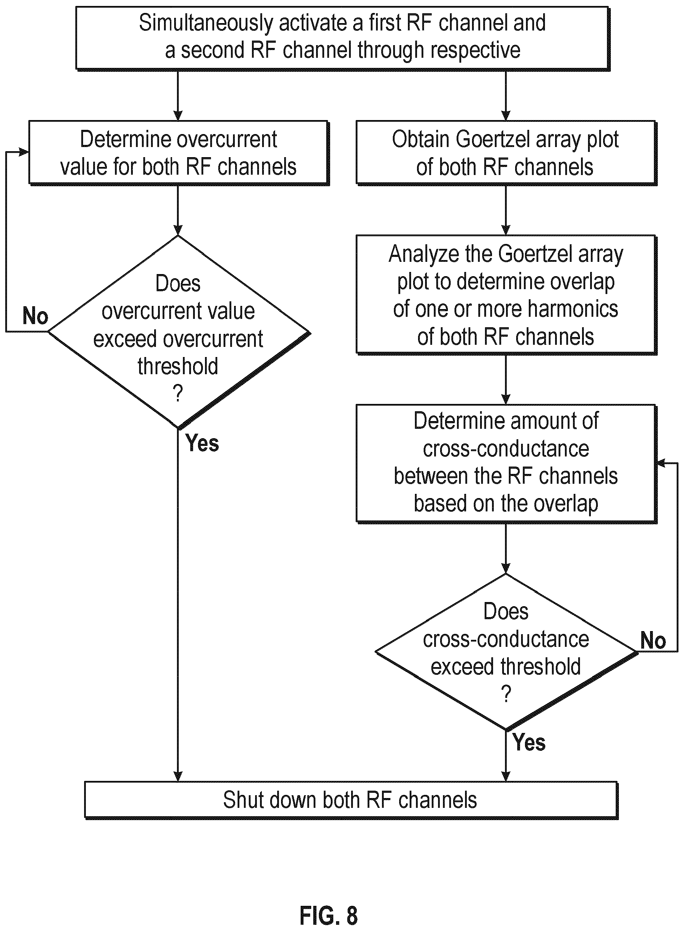

With reference to FIG. 8, a method of monitoring output of the first RF source 202 and the RF second source 302, including interrogation waveform and RF bipolar waveforms for overcurrent and cross-conductance. Once the processors 204 and 304 confirm that the instruments 20' and 20" are in contact with tissue, the processors 204 and 304 signal the first RF source 202 and/or the RF second source 302 to output first and second bipolar RF waveforms to treat tissue, depending on which of the instruments 20' or 20" contacted tissue. Thus, if only one of the instruments 20' or 20" was confirmed to have contacted tissue based on the detection method of FIG. 7, only that corresponding first or second RF source 202 or 302 is energized to output the bipolar waveform to treat tissue. The other one of the first or second RF source 202 or 302 remains in an interrogation mode until the user exits the auto bipolar mode or tissue contact is detected.

The first and second bipolar RF waveforms may be supplied simultaneously, if both interrogation waveforms confirm tissue contact. During simultaneous bipolar RF waveform transmission, the sensors 216 and 316 measure properties of the first and second RF waveforms. Bipolar RF waveform carrier frequencies and coagulation repetition rates may be chosen to provide coherent sampling under time critical power computation update rates.

The sensors 216 and 316 in conjunctions with the respective controllers 204 and 304 perform a wide band measurement of each first and second RF waveforms while at the same time detecting cross-conductance between the first RF source 202 and the second RF source 302.

Each of the controllers 204 and 304 also utilize a signal processing technique, namely, the Goertzel array plots described above, to discriminate between simultaneously activated the first RF source 202 and the second RF source 302. The controllers 204 and 304 are also configured to determine a level of cross-conductance between first RF source 202 and the second RF source 302, if any, using the signal processing discrimination technique. After the level of cross-conductance is determined, the level of cross-conductance is used as a safety mitigator, namely, as a dosage error monitor, during simultaneous bipolar RF operation. In particular, upon detection of cross-conductance by either of the controllers 204 or 304, each of the controllers 204 and 304 is configured to output an alarm and/or shut down both the first RF source 202 and the second RF source 302 in response to the cross-conductance dosage error.

While several embodiments of the disclosure have been shown in the drawings and/or described herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

The invention may be described by reference to the following numbered paragraphs:-
1. An electrosurgical generator comprising:
   a first radio frequency source including:
      a first power supply configured to output a first direct current waveform;
      a first radio frequency inverter coupled to the first power supply and configured to generate a first interrogation waveform and a first radio frequency waveform from the first direct current waveform; and
      a first controller configured to control the first radio frequency inverter to output the first radio frequency waveform based on a response of the first interrogation waveform; and
   a second radio frequency source including:
      a second power supply configured to output a second direct current waveform;
      a second radio frequency inverter coupled to the second power supply and configured to generate a second interrogation waveform simultaneously as the first interrogation waveform and a second radio frequency waveform simultaneously as the first radio frequency waveform; and
      a second controller configured to control the second radio frequency inverter to output the second radio frequency waveform based on a response of the second interrogation waveform.
2. The electrosurgical generator according to paragraph 1, wherein each of the first interrogation waveform and the second interrogation waveform is a pulsed radio frequency waveform.
3. The electrosurgical generator according to paragraph 2, wherein the pulsed radio frequency waveform includes a plurality of pulses having a duration from about 10 µsec to about 1,000 µsec and having a repetition rate of from about 10 msec to about 50 msec.
4. The electrosurgical generator according to paragraph 1, wherein the first radio frequency source further includes at least one first sensor configured to measure a first impedance based the first interrogation waveform.
5. The electrosurgical generator according to paragraph 4, wherein the first controller is further configured to determine contact between a first electrosurgical instrument coupled to the first radio frequency source and tissue based on a comparison of the first impedance to an open circuit threshold.
6. The electrosurgical generator according to paragraph 5, wherein the first controller is further configured to control the first radio frequency inverter to output the first radio frequency waveform based on the comparison.
7. The electrosurgical generator according to paragraph 6, wherein the second radio frequency source further includes at least one second sensor configured to measure a second impedance based on the second interrogation waveform.
8. The electrosurgical generator according to paragraph 7, wherein the second controller is further configured to determine contact between a second electrosurgical instrument coupled to the second radio frequency source and tissue based on a comparison of the second impedance to an open circuit threshold.
9. The electrosurgical generator according to paragraph 8, wherein the second controller is further configured to control the second radio frequency inverter to output the second radio frequency waveform based on the comparison.
10. The electrosurgical generator according to paragraph 1, further comprising:
   a clock source coupled to the first controller and the second controller and configured to synchronize operation of the first controller and the second controller.
11. An electrosurgical system comprising:
   a first bipolar electrosurgical instrument;
   a second bipolar electrosurgical instrument; and
   an electrosurgical generator including:
      a first radio frequency source including:
         a first power supply configured to output a first direct current waveform;
         a first radio frequency inverter coupled to the first power supply and the first bipolar electrosurgical instrument, the first radio frequency inverter configured to generate a first interrogation waveform and a first radio frequency waveform from the first direct current waveform; and
         a first controller configured to control the first radio frequency inverter to output the first radio frequency waveform based on a response of the first interrogation waveform; and
      a second radio frequency source including:
         a second power supply configured to output a second direct current waveform;
         a second radio frequency inverter coupled to the second power supply and the second bipolar electrosurgical instrument, the second radio frequency inverter configured to generate a second interrogation waveform simultaneously as the first interrogation waveform and a second radio frequency waveform simultaneously as the first radio frequency waveform; and
         a second controller configured to control the second radio frequency inverter to output the second radio frequency waveform based on a response of the second interrogation waveform.
12. The electrosurgical system according to paragraph 11, wherein each of the first interrogation waveform and the second interrogation waveform is a pulsed radio frequency waveform.
13. The electrosurgical system according to paragraph 12, wherein the pulsed radio frequency waveform includes a plurality of pulses having a duration from about 10 µsec to about 1,000 µsec and having a repetition rate of from about 10 msec to about 50 msec.
14. The electrosurgical system according to paragraph 11, wherein the first radio frequency source further includes at least one first sensor configured to measure a first impedance based the first interrogation waveform.
15. The electrosurgical system according to paragraph 14, wherein the first controller is further configured to determine contact between the first bipolar electrosurgical instrument coupled to the first radio frequency source and tissue based on a comparison of the first impedance to an open circuit threshold.
16. The electrosurgical system according to paragraph 15, wherein the first controller is further configured to control the first radio frequency inverter to output the first radio frequency waveform based on the comparison.
17. The electrosurgical system according to paragraph 16, wherein the second radio frequency source further includes at least one second sensor configured to measure a second impedance based on the second interrogation waveform.
18. The electrosurgical system according to paragraph 17, wherein the second controller is further configured to determine contact between the second bipolar electrosurgical instrument coupled to the second radio frequency source and tissue based on a comparison of the second impedance to an open circuit threshold.
19. The electrosurgical system according to paragraph 18, wherein the second controller is further configured to control the second radio frequency inverter to output the second radio frequency waveform based on the comparison.
20. The electrosurgical system according to paragraph 11, wherein the electrosurgical generator further includes a clock source coupled to the first controller and the second controller and configured to synchronize operation of the first controller and the second controller.

## Claims

1. An electrosurgical generator comprising:
a first radio frequency source including:
a first power supply configured to output a first direct current waveform;
a first radio frequency inverter coupled to the first power supply and configured to generate a first interrogation waveform and a first radio frequency waveform from the first direct current waveform; and
a first controller configured to control the first radio frequency inverter to output the first radio frequency waveform based on a response of the first interrogation waveform; and
a second radio frequency source including:
a second power supply configured to output a second direct current waveform;
a second radio frequency inverter coupled to the second power supply and configured to generate a second interrogation waveform simultaneously as the first interrogation waveform and a second radio frequency waveform simultaneously as the first radio frequency waveform; and
a second controller configured to control the second radio frequency inverter to output the second radio frequency waveform based on a response of the second interrogation waveform.

2. The electrosurgical generator according to claim 1, wherein each of the first interrogation waveform and the second interrogation waveform is a pulsed radio frequency waveform.

3. The electrosurgical generator according to claim 2, wherein the pulsed radio frequency waveform includes a plurality of pulses having a duration from about 10 µsec to about 1,000 µsec and having a repetition rate of from about 10 msec to about 50 msec.

4. The electrosurgical generator according to claim 1, 2 or 3 wherein the first radio frequency source further includes at least one first sensor configured to measure a first impedance based the first interrogation waveform.

5. The electrosurgical generator according to claim 4, wherein the first controller is further configured to determine contact between a first electrosurgical instrument coupled to the first radio frequency source and tissue based on a comparison of the first impedance to an open circuit threshold.

6. The electrosurgical generator according to claim 5, wherein the first controller is further configured to control the first radio frequency inverter to output the first radio frequency waveform based on the comparison.

7. The electrosurgical generator according to claim 6, wherein the second radio frequency source further includes at least one second sensor configured to measure a second impedance based on the second interrogation waveform.

8. The electrosurgical generator according to claim 7, wherein the second controller is further configured to determine contact between a second electrosurgical instrument coupled to the second radio frequency source and tissue based on a comparison of the second impedance to an open circuit threshold.

9. The electrosurgical generator according to claim 8, wherein the second controller is further configured to control the second radio frequency inverter to output the second radio frequency waveform based on the comparison.

10. The electrosurgical generator according to any preceding claim, further comprising:
a clock source coupled to the first controller and the second controller and configured to synchronize operation of the first controller and the second controller.

11. An electrosurgical system comprising:
a first bipolar electrosurgical instrument;
a second bipolar electrosurgical instrument; and
an electrosurgical generator as claimed in any preceding claim.
